# FASCICULE DE BREVET EUROPEEN

(11) **EP 2 197 791 B1**
(45) Date de publication et mention de la délivrance du brevet: **19.10.2011**
(21) Numéro de dépôt: 08845167.9
(22) Date de dépôt: 18.08.2008
(51) Int. Cl.: C01B 37/00, C01B 37/02, C01B 39/00, B01J 29/00, B01J 37/00, C01B 33/113, B01J 21/06, B01J 29/06

(54) **MATERIAU AMORPHE A POROSITE HIERARCHISEE ET COMPRENANT DU SILICIUM**
AMORPHES MATERIAL MIT HIERARCHISIERTER POROSITÄT UND MIT SILICIUM
AMORPHOUS MATERIAL HAVING A HIERARCHISED POROSITY AND COMPRISING SILICON

(30) Priorité: 07.09.2007 FR 0706296
(43) Date de publication de la demande: 23.06.2010
(73) Titulaire: IFP Energies nouvelles, 92852 Rueil-Malmaison Cedex (FR)
(72) Inventeur: CHAUMONNOT, Alexandra, F-69008 Lyon (FR); COUPE, Aurélie, F-77186 Noisiel. (FR); SANCHEZ, Clément, F-91190 Gif-sur-yvette (FR); BOISSIERE, Cédric, F-75013 Paris (FR)
(86) Numéro de dépôt international: PCT/FR2008/001198
(87) Numéro de publication internationale: WO 2009/056710

(56) Documents cités:
- EP-A- 1 627 853
- TAKAHASHI R ET AL: "Synthesis of monolithic zeolites with macropores" JOURNAL OF THE CERAMIC SOCIETY OF JAPAN CERAMIC SOC. JAPAN JAPAN, vol. 114, no. 5, 1 mai 2006 (2006-05-01), pages 421-424, XP002414823 ISSN: 0914-5400
- LI WEN-CUI ET AL: "Hierarchically structured monolithic silicalite-1 consisting of crystallized nanoparticles and its performance in the Beckmann rearrangement of cyclohexanone oxime" JOURNAL OF THE AMERICAN CHEMICAL SOCIETY SEP 14 2005, vol. 127, no. 36, 14 septembre 2005 (2005-09-14), pages 12595-12600, XP002414824
- VALTCHEV VALENTIN P ET AL: "Equisetum arvense Templating of Zeolite Beta Macrostructures with Hierarchical Porosity" CHEM. MATER.; CHEMISTRY OF MATERIALS APR 6 2004, vol. 16, no. 7, 6 avril 2004 (2004-04-06), pages 1350-1355, XP002414831
- BAOJIAN ZHANG ET AL: "Starch gel templating of spongelike macroporous silicalite monoliths and mesoporous films" CHEMISTRY OF MATERIALS AMERICAN CHEM. SOC USA, vol. 14, no. 3, mars 2002 (2002-03), pages 1369-1375, XP002414825 ISSN: 0897-4756
- GROEN JOHAN C ET AL: "Mechanism of hierarchical porosity development in MFI zeolites by desilication: The role of aluminium as a pore-directing agent" CHEM. EUR. J.; CHEMISTRY - A EUROPEAN JOURNAL AUG 19 2005, vol. 11, no. 17, 19 août 2005 (2005-08-19), pages 4983-4994, XP002414826
- NAYDENOV VALERI ET AL: "Self-bonded zeolite beta/MCM-41 composite spheres" J POROUS MATER; JOURNAL OF POROUS MATERIALS JULY 2005, vol. 12, no. 3, juillet 2005 (2005-07), pages 193-199, XP002414827
- HUANG L ET AL: "Hierarchical porous structures by using zeolite nanocrystals as building blocks" MICROPOROUS MESOPOROUS MATER.; MICROPOROUS AND MESOPOROUS MATERIALS NOVEMBER 2001, vol. 48, no. 1-3, novembre 2001 (2001-11), pages 73-78, XP002414828

## Description

La présente invention se rapporte au domaine des matériaux comprenant du silicium, notamment des matériaux métallosilicates et plus précisément des matériaux aluminosilicates, présentant une porosité hiérarchisée dans le domaine de la microporosité, de la mésoporosité et de la macroporosité. Elle concerne également la préparation de ces matériaux qui sont obtenus par l'emploi de la technique de synthèse dite "aérosol".

### Etat de la technique antérieure

Les nouvelles stratégies de synthèse permettant d'obtenir des matériaux à porosité bien définie dans une très large gamme, allant des matériaux microporeux aux matériaux macroporeux en passant par des matériaux à porosité hiérarchisée, c'est-à-dire ayant des pores de plusieurs tailles, connaissent un très large développement au sein de la communauté scientifique depuis le milieu des années 90 (G. J. de A. A. Soler-Illia, C. Sanchez, B. Lebeau, J. Patarin, Chem. Rev., 2002, 102, 4093). En particulier, de nombreux travaux portent sur le développement de matériaux présentant une microporosité de nature zéolithique et une mésoporosité de façon à bénéficier simultanément des propriétés catalytiques propres aux zéolithes et des propriétés catalytiques et surtout texturales de la phase mésoporeuse.

Une technique fréquemment employée pour générer des matériaux présentant cette biporosité consiste à créer directement au sein de cristaux de zéolithe des mésopores en faisant subir à cette dernière un traitement hydrothermal sous vapeur d'eau encore appelé "steaming". Sous l'effet de ce traitement, la mobilité des atomes tétraédriques qui composent la charpente de la zéolithe est accrue au point que certains de ces atomes sont extraits du réseau, ce qui engendre la formation de zone amorphe, qui peuvent être dégagés pour laisser la place à des cavités mésoporeuses (A. H. Jansen, A. J. Koster, K. P. De Jong, J. Phys. Chem. B, 2002, 106, 11905). La formation de telles cavités peut aussi être obtenue par traitement acide de la zéolithe (H. Ajot, J. F. Joly, J. Lynch, F. Raatz, P. Caullet, Stud. Surf. Sci. Catal., 1991, 62, 583). Ces méthodes présentent toutefois l'inconvénient de rendre partiellement amorphe une partie de la zéolithe et de modifier ses propriétés par variation de la composition chimique. Dans tous les cas, la mésoporosité ainsi introduite permet d'éliminer ou tout du moins de limiter les problèmes de limitations diffusionnelles rencontrés dans des matériaux microporeux, les mésopores présentant des facteurs de diffusion bien plus importants que les micropores et permettant ainsi l'accès aux sites actifs de la zéolithe (P. B. Weisz, Chemtech, 1973, 3, 498).
Plus récemment, de nombreux travaux ont porté sur l'élaboration de matériaux mixtes mésostructuré/zéolithe, les matériaux mésostructurés présentant l'avantage supplémentaire d'avoir une porosité parfaitement organisée et calibrée dans la gamme des mésopores.

Nous rappelons ici brièvement que les matériaux mésostructurés sont classiquement obtenus via des méthodes de synthèse dites de chimie douce qui consistent à mettre en présence en solution aqueuse ou dans des solvants polaires de précurseurs inorganiques avec des agents structurants, généralement des tensioactifs moléculaires ou macromoléculaires, ioniques ou neutres. Le contrôle des interactions électrostatiques ou par liaisons hydrogènes entre les précurseurs inorganiques et l'agent structurant conjointement lié à des réactions d'hydrolyse/condensation du précurseur inorganique conduit à un assemblage coopératif des phases organique et inorganique générant des agrégats micellaires de tensioactifs de taille uniforme et contrôlée au sein d'une matrice inorganique. La libération de la porosité est ensuite obtenue par élimination du tensioactif, celle-ci étant réalisée classiquement par des procédés d'extraction chimique ou par traitement thermique. En fonction de la nature des précurseurs inorganiques et de l'agent structurant employé ainsi que des conditions opératoires imposées, plusieurs familles de matériaux mésostructurés ont été développées comme la famille des M41S obtenue via l'emploi de sels d'ammonium quaternaire comme agent structurant (J. S. Beck, J. C. Vartuli, W. J. Roth, M. E. Leonowicz, C. T. Kresge, K. D. Schmitt, C. T.-W. Chu, D. H. Olson, E. W. Sheppard, S. B. McCullen, J. B. Higgins, J. L. Schlenker, J. Am. Chem. Soc., 1992, 114, 27, 10834) ou bien la famille des SBA obtenue via l'emploi de copolymères triblocs comme agents structurants (D. Zhao, J. Feng, Q. Huo, N. Melosh, G. H. Fredickson, B. F. Chmelka, G. D. Stucky, Science, 1998, 279, 548).

Plusieurs techniques de synthèse permettant l'élaboration de ces matériaux mixtes mésostructuré/zéolithe ont ainsi été répertoriées dans la littérature ouverte. Une première technique de synthèse consiste à synthétiser en première étape un matériau aluminosilicate mésostructuré selon les méthodes classiques explicitées ci-dessus puis, en deuxième étape, à imprégner ce matériau avec un agent structurant habituellement utilisé dans la synthèse de matériaux zéolithiques. Un traitement hydrothermique adapté conduit à une zéolithisation des parois (ou murs) amorphes de l'aluminosilicate mésostructuré de départ (K. R. Koletstra, H. van Bekkum, J. C. Jansen, Chem. Commun., 1997, 2281 ; D. T. On, S. Kaliaguine, Angew. Chem. Int. Ed., 2001, 40, 3248 ; D. T. On, D. Lutic, S. Kaliaguine, Micropor. Mesopor. Mater., 2001, 44, 435 ; M. J. Verhoef, P. J. Kooyman, J. C. van der Waal, M. S. Rigutto, J. A. Peters, H. van Bekkum, Chem. Mater., 2001, 13, 683 ; US 6.669.924). Une deuxième technique de synthèse consiste cette fois-ci à mettre en présence une solution colloïdale de germes de zéolithes avec un tensioactif habituellement utilisé pour créer une mésostructuration du matériau final. L'idée mise à profit ici est de générer simultanément l'élaboration d'une matrice inorganique à mésoporosité organisée et la croissance au sein de cette matrice des germes de zéolithes de façon à obtenir idéalement un matériau aluminosilicate mésostructuré présentant des parois (ou murs) cristallisées (Z. Zhang et al, J. Am. Chem. Soc., 2001, 123, 5014 ; Y. Liu et al, J. Am. Chem., Soc., 2000, 122, 8791). Une variante à ces deux techniques consiste à partir d'un mélange de précurseurs d'aluminium et de silicium en présence de deux agents structurants, l'un susceptible de générer un système zéolithique, l'autre susceptible de générer une mésostructuration. Cette solution est ensuite soumise à deux étapes de cristallisation à l'aide de conditions de traitement hydrothermal variables, une première étape qui conduit à la formation de la structure mésoporeuse à porosité organisée et une deuxième étape qui conduit à la zéolithisation des parois (ou murs) amorphes (A. Karlsson, M. Stôcker, R. Schmidt, Micropor. Mesopor. Mater., 1999, 27, 181 ; L. Huang, W. Guo, P. Deng, Z. Xue, Q. Li, J. Phys., Chem. B, 2000, 104, 2817. L'ensemble de ces méthodes de synthèse présente l'inconvénient d'endommager la mésostructure et donc de perdre les avantages de celle-ci dans le cas où la croissance des germes de zéolithes ou la zéolithisation des parois n'est pas parfaitement maîtrisée, ce qui rend ces techniques délicates à mettre en oeuvre.

Nous notons qu'il est également possible d'élaborer directement des matériaux composites mésostructuré/zéolithe de façon à bénéficier des propriétés catalytiques propres à chacune de ces phases. Ceci peut se faire par traitement thermique d'un mélange d'une solution de germes de zéolithes et d'une solution de germes d'aluminosilicates mésostructurés (P. Prokesova, S. Mintova, J. Cejka, T. Bein, Micropor. Mesopor. Mater., 2003, 64, 165) ou bien par croissance d'une couche de zéolithe à la surface d'un aluminosilicate mésostructuré présynthétisé (D. T. On, S. Kaliaguine, Angew. Chem. Int. Ed., 2002, 41, 1036).

En excluant les matériaux zéolithiques mésoporeux obtenus par post-traitement d'une zéolithe, nous notons que, d'un point de vue expérimental, tous ces matériaux sont obtenus par précipitation directe de précurseurs inorganiques en présence ou non d'agents structurants au sein d'une solution aqueuse ou dans des solvants polaires, cette étape étant la plupart du temps suivie par une ou plusieurs étapes de mûrissement en autoclave. Les particules élémentaires habituellement obtenues ne présentent pas de forme régulière et sont caractérisées généralement par une taille variant de 200 à 500 nm.

Plusieurs travaux ont également porté sur l'élaboration de matériaux présentant à la fois une microporosité et une macroporosité. A titre d'exemple, une des méthodes de synthèse les plus courantes consiste à utiliser des billes de polystyrène comme élément générateur de la macroporosité et à créer autour de ces billes un réseau zéolithique (G. S. Zhu, S. L. Qiu, F. F. Gao, D. S. Li, Y. F. Li, R. W. Wang, B. Gao, B. S. Li, Y. H. Guo, R. R. Xu, Z. Liu, O. Terasaki, J. Mater. Chem., 2001, 11, 6, 1687).

Le document EP1627853 décrit un matériau à porosité hiérarchisée constitué d'au moins deux particules sphériques élémentaires, chacune desdites particules sphériques comprenant des nanocristaux zéolithiques ayant une taille de pores comprise entre 0,2 et 2 nm et une matrice à base d'oxyde de silicium, mésostructurée, ayant une taille de pores comprise entre 1,5 et 30 nm et présentant des parois amorphes d'épaisseur comprise entre 1 et 20 nm, lesdites particules sphériques élémentaires ayant un diamètre maximal de 10 microns.

### Résumé de l'invention

L'invention concerne un matériau à porosité hiérarchisée constitué d'au moins deux particules sphériques élémentaires ayant un diamètre maximal de 200 microns, l'une au moins desdites particules sphériques comprend au moins une matrice à base d'oxyde de silicium, ledit matériau présentant un volume macroporeux mesuré par porosimétrie au mercure compris entre 0,05 et 1 ml/g, un volume mésoporeux mesuré par volumétrie à l'azote compris entre 0,01 et 1 ml/g et un volume microporeux mesuré par volumétrie à l'azote compris entre 0,03 et 0,4 ml/g, ladite matrice présentant des parois amorphes, lesquelles sont constituées de germes zéolithiques à l'origine de la microporosité du matériau.

Ladite matrice à base d'oxyde de silicium comprend éventuellement, en outre, au moins un élément X choisi parmi l'aluminium, le fer, le bore, l'indium et le gallium, de préférence l'aluminium. La présente invention concerne également la préparation du matériau selon l'invention. Le procédé de préparation du matériau selon l'invention comprend : a) la préparation d'une solution limpide contenant les éléments précurseurs de germes zéolithiques, à savoir au moins un agent structurant, au moins un précurseur silicique et éventuellement au moins un précurseur d'au moins un élément X choisi parmi l'aluminium, le fer, le bore, l'indium et le gallium ; b) le mélange en solution d'au moins un tensioactif et d'au moins ladite solution limpide obtenue selon a) ; c) l'atomisation par aérosol de ladite solution obtenue à l'étape b) pour conduire à la formation de gouttelettes sphériques ; d) le séchage desdites gouttelettes et e) l'élimination dudit agent structurant et dudit tensioactif pour l'obtention d'un matériau amorphe à porosité hiérarchisée dans la gamme de la microporosité, de la mésoporosité et de la macroporosité.

La microporosité induite par les parois amorphes du matériau selon l'invention est consécutive non seulement à l'emploi d'une solution contenant les éléments précurseurs de germes zéolithiques conformément à l'étape a) du procédé selon l'invention mais également à l'atomisation par aérosol de la solution comprenant au moins un tensioactif et une solution limpide conformément à l'étape c) du procédé selon l'invention. La mésoporosité et la macroporosité du matériau selon l'invention sont consécutives au phénomène de séparation de phase par décomposition spinodale de la phase organique générée par la présence du tensioactif et de la phase inorganique issue de la solution contenant les éléments précurseurs des germes zéolithiques, ce phénomène de séparation de phase étant induit par la technique dite aérosol conformément à l'étape c) du procédé selon l'invention.

### Intérêt de l'invention

Le matériau selon l'invention qui comprend une matrice inorganique mésoporeuse et macroporeuse, à base d'oxyde de silicium, aux parois microporeuses et amorphes présente simultanément les propriétés texturales propres aux matériaux microporeux, aux matériaux mésoporeux et aux matériaux macroporeux. De manière préférée, la matrice à base d'oxyde de silicium formant chacune des particules sphériques élémentaires du matériau selon l'invention comprend, outre du silicium, au moins un élément X choisi parmi l'aluminium, le fer, l'indium et le gallium, préférentiellement l'aluminium de manière à former une matrice aluminosilicate amorphe. Le matériau selon l'invention présente alors, lorsque X est l'aluminium, des propriétés d'acido-basicité supérieures aux propriétés d'acido-basicité présentées par les matériaux aluminosilicates amorphes antérieurs, dépourvus de précurseurs zéolithiques, et préparés selon des protocoles de synthèse bien connus de l'Homme du métier utilisant des précurseurs inorganiques de silice et d'alumine. Par ailleurs, la présence au sein d'une même particule sphérique de taille micrométrique voire nanométrique de mésopores et de macropores dans une matrice inorganique microporeuse et amorphe conduit à un accès privilégié des réactifs et des produits de la réaction aux sites microporeux lors de l'emploi du matériau selon l'invention en tant qu'adsorbant ou que solide acide dans des applications industrielles potentielles. De plus, le matériau selon l'invention est constitué de particules élémentaires sphériques, le diamètre de ces particules étant au maximum égal à 200 µm, de préférence inférieur à 100 µm, variant avantageusement de 50 nm à 20 µm, très avantageusement de 50 nm à 10 µm et de manière encore plus avantageuse de 50 à 300 nm. La taille limitée de ces particules ainsi que leur forme sphérique homogène permet d'avoir une meilleure diffusion des réactifs et des produits de la réaction lors de l'emploi du matériau selon l'invention dans des applications industrielles potentielles comparativement à des matériaux connus de l'état de la technique se présentant sous la forme de particules élémentaires de forme non homogène, c'est-à-dire irrégulière, et de taille souvent supérieure à 500 nm.

### Exposé de l'invention

La présente invention a pour objet un matériau à porosité hiérarchisée constitué d'au moins deux particules sphériques élémentaires ayant un diamètre maximal de 200 microns, l'une au moins desdites particules sphériques comprend au moins une matrice à base d'oxyde de silicium et ayant des parois amorphes, ledit matériau présentant un volume macroporeux mesuré par porosimétrie au mercure compris entre 0,05 et 1 ml/g, un volume mésoporeux mesuré par volumétrie à l'azote compris entre 0,01 et 1 ml/g et un volume microporeux mesuré par volumétrie à l'azote compris entre 0,03 et 0,4 ml/g.

Par matériau à porosité hiérarchisée, on entend au sens de la présente invention un matériau présentant au moins une, et généralement plusieurs, particule(s) sphérique(s) ayant une triple porosité : une macroporosité caractérisée par un volume mercure macroporeux compris dans une gamme variant de 0,05 à 1 ml/g et de préférence dans une gamme variant de 0,1 à 0,3 ml/g, une mésoporosité caractérisée par un volume mésoporeux mesuré par volumétrie à l'azote compris dans une gamme variant de 0,01 à 1 ml/g et de préférence dans une gamme variant de 0,1 à 0,6 ml/g et une microporosité induite par les parois amorphes, les caractéristiques de la microporosité étant fonction des germes zéolithiques constitutifs des parois amorphes de la matrice de chacune des particules sphériques du matériau selon l'invention. La macroporosité est également caractérisée par la présence de domaines macroporeux compris dans une gamme variant de 50 à 1000 nm et de préférence dans une gamme variant de 80 à 500 nm et/ou résulte d'une macroporosité texturale intraparticulaire, la mésoporosité est aussi caractérisée par la présence de domaines mésoporeux compris dans une gamme variant de 2 à 50 nm et de préférence dans une gamme variant de 10 à 50 nm. Le matériau selon l'invention peut également avantageusement présenter des particules sphériques élémentaires dépourvues de mésoporosité. II est à noter qu'une porosité de nature microporeuse peut également résulter de l'imbrication du tensioactif, utilisé lors de la préparation du matériau selon l'invention, avec la paroi inorganique au niveau de l'interface organique-inorganique développée lors de l'élaboration dudit matériau selon l'invention.

Conformément à l'invention, la matrice à base d'oxyde de silicium formant chacune des particules sphériques du matériau selon l'invention présente des parois amorphes constituées de germes zéolithiques, lesquels sont à l'origine de la microporosité présente au sein de chacune des particules sphériques du matériau selon l'invention. Les germes zéolithiques sont des espèces préparées à partir de réactifs utilisés pour la synthèse de zéolithes, la préparation desdites espèces n'ayant pas été menée jusqu'au stade de la formation de zéolithes cristallisées. Il en résulte que lesdits germes, de petite taille, ne sont pas détectés lorsqu'ils sont caractérisés par diffraction aux rayons X aux grands angles. Plus précisément et conformément à l'invention, les germes zéolithiques constituant les parois microporeuses amorphes de la matrice de chacune des particules sphériques du matériau selon l'invention sont des espèces pouvant servir d'amorce à la synthèse de toute zéolithe connue de l'Homme du métier et en particulier, mais de façon non exhaustive, à la synthèse des zéolithes répertoriées dans l"Atlas of zeolite framework types", 5th revised Edition, 2001, C. Baerlocher, W. M. Meier, D. H. Olson, la mise en solution des éléments précurseurs des germes zéolithiques, à savoir au moins un agent structurant, au moins un précurseur silicique et éventuellement au moins un précurseur d'au moins un élément X choisi parmi l'aluminium, le fer, le bore, l'indium et le gallium, de préférence l'aluminium, étant telle qu'elle conduise à l'obtention d'une solution limpide. Les germes zéolithiques constitutifs des parois amorphes de la matrice de chacune des particules du matériau selon l'invention et à l'origine de la microporosité de celui-ci sont de préférence des espèces pour l'amorce d'au moins une zéolithe choisie parmi les zéolithes ZSM-5, ZSM-48, ZSM-22, ZSM-23, ZBM-30, EU-2, EU-11, Silicalite, Bêta, zéolithe A, Faujasite, Y, USY, VUSY, SDUSY, mordénite, NU-87, NU-88, NU-86, NU-85, IM-5, IM-12, Ferriérite et EU-1. De manière très préférée, les germes zéolithiques constituant les parois amorphes de la matrice de chacune des particules du matériau selon l'invention sont des espèces pour l'amorce d'au moins une zéolithe choisie parmi les zéolithes de type structural MFI, BEA, FAU et LTA.

Conformément à l'invention, la matrice à base d'oxyde de silicium formant chacune des particules sphériques élémentaires du matériau selon l'invention est soit entièrement silicique soit, elle comprend, outre du silicium, au moins un élément X choisi parmi l'aluminium, le fer, le bore, l'indium et le gallium, de préférence l'aluminium. Ainsi, les germes zéolithiques constitutifs des parois amorphes de la matrice de chacune des particules sphériques du matériau selon l'invention et à l'origine de la microporosité de celui-ci sont avantageusement des espèces pour l'amorce d'au moins une zéolithe soit entièrement silicique soit contenant, outre du silicium, au moins un élément X choisi parmi l'aluminium, le fer, le bore, l'indium et le gallium, de préférence l'aluminium. Lorsque X est l'aluminium, la matrice du matériau est un aluminosilicate amorphe, précurseur d'un matériau aluminosilicate cristallisé. Cet aluminosilicate amorphe présente un rapport molaire Si/Al égal à celui de la solution des précurseurs silicique et aluminique conduisant à la formation des germes zéolithiques constituant les parois amorphes de la matrice. Lesdites particules sphériques élémentaires constituant le matériau selon l'invention sont dépourvues de nanocristaux zéolithiques.

Conformément à l'invention, lesdites particules sphériques élémentaires constituant le matériau selon l'invention ont un diamètre maximal égal à 200 microns, de préférence inférieur à 100 microns, avantageusement compris entre 50 nm et 20 µm, très avantageusement compris entre 50 nm et 10 µm, et de manière encore plus avantageuse compris entre 50 et 300 nm. Plus précisément, elles sont présentes dans le matériau selon l'invention sous la forme d'agrégats.

Le matériau selon l'invention présente avantageusement une surface spécifique comprise entre 100 et 1100 m²/g et de manière très avantageuse comprise entre 200 et 800 m²/g.

La présente invention a également pour objet la préparation du matériau selon l'invention. Ledit procédé de préparation du matériau selon l'invention comprend : a) la préparation d'une solution limpide contenant les éléments précurseurs de germes zéolithiques, à savoir au moins un agent structurant, au moins un précurseur silicique et éventuellement au moins un précurseur d'au moins un élément X choisi parmi l'aluminium, le fer, le bore, l'indium et le gallium, de préférence l'aluminium ; b) le mélange en solution d'au moins un tensioactif et d'au moins ladite solution limpide obtenue selon a) ; c) l'atomisation par aérosol de ladite solution obtenue à l'étape b) pour conduire à la formation de gouttelettes sphériques ; d) le séchage desdites gouttelettes et e) l'élimination dudit agent structurant et dudit tensioactif pour l'obtention d'un matériau amorphe à porosité hiérarchisée dans la gamme de la microporosité, de la mésoporosité et de la macroporosité.

Conformément à l'étape a) du procédé de préparation selon l'invention, la solution limpide contenant les éléments précurseurs de germes zéolithiques, à savoir au moins un agent structurant, au moins un précurseur silicique et éventuellement au moins un précurseur d'au moins un élément X choisi parmi l'aluminium, le fer, le bore, l'indium et le gallium, de préférence l'aluminium, est réalisée à partir de protocoles opératoires connus de l'Homme du métier.

Le précurseur silicique utilisé pour la mise en oeuvre de l'étape a) du procédé selon l'invention est choisi parmi les précurseurs d'oxyde de silicium bien connus de l'Homme du métier. En particulier, on utilise avantageusement un précurseur silicique choisi parmi les précurseurs de silice habituellement utilisés dans la synthèse des zéolithes, par exemple on utilise de la silice solide en poudre, de l'acide silicique, de la silice colloïdale, de la silice dissoute ou du tétraéthoxysilane encore appelé tétraéthylortosilicate (TEOS). De manière préférée, le précurseur silicique est le TEOS.

Le précurseur de l'élément X, éventuellement utilisé pour la mise en oeuvre de l'étape a) du procédé selon l'invention, peut être tout composé comprenant l'élément X et pouvant libérer cet élément en solution, notamment en solution aqueuse ou aquo-organique, sous forme réactive. Dans le cas préféré où X est l'aluminium, le précurseur aluminique est avantageusement un sel inorganique d'aluminium de formule AlZ₃, Z étant un halogène, un nitrate ou un hydroxyde. De préférence, Z est le chlore. Le précurseur aluminique peut également être un sulfate d'aluminium de formule Al₂(SO₄)₃. Le précurseur aluminique peut être aussi un précurseur organométallique de formule Al(OR)₃ ou R = éthyle, isopropyle, n-butyle, s-butyle (Al(O^{s}C₄H₉)₃) ou t-butyle ou un précurseur chélaté tel que l'aluminium acétylacétonate (Al(C₅H₈O₂)₃). De préférence, R est le s-butyle. Le précurseur aluminique peut aussi être de l'aluminate de sodium ou de l'alumine proprement dite sous l'une de ses phases cristallines connues de l'Homme du métier (alpha, delta, teta, gamma), de préférence sous forme hydratée ou qui peut être hydratée.

On peut également utiliser des mélanges des précurseurs cités ci-dessus. Certains ou l'ensemble des précurseurs aluminiques et siliciques peuvent éventuellement être ajoutés sous la forme d'un seul composé comprenant à la fois des atomes d'aluminium et des atomes de silicium, par exemple une silice alumine amorphe.

L'agent structurant utilisé pour la mise en oeuvre de l'étape a) du procédé selon l'invention peut être ionique ou neutre selon la nature de la zéolithe obtenue à partir desdits germes zéolithiques. Il est fréquent d'utiliser les agents structurants de la liste non exhaustive suivante : des cations organiques azotés tel que le tétrapropylammonium (TPA), des éléments de la famille des alcalins (Cs, K, Na, etc.), des éthercouronnes, des diamines ainsi que tout autre agent structurant bien connu de l'Homme de l'Art pour la synthèse de zéolithe.

On obtient généralement la solution limpide contenant les éléments précurseurs de germes zéolithiques selon l'étape a) du procédé de préparation du matériau selon l'invention en préparant un mélange réactionnel renfermant au moins un précurseur silicique, éventuellement au moins un précurseur d'au moins un élément X choisi parmi l'aluminium, le fer, le bore, l'indium et le gallium, de préférence au moins un précurseur aluminique, et au moins un agent structurant. Le mélange réactionnel est soit aqueux soit aquo-organique, par exemple un mélange eau-alcool. Il est préféré de travailler dans un milieu réactionnel basique au cours des diverses étapes du procédé selon l'invention afin de favoriser le développement des germes zéolithiques constitutifs des parois amorphes de la matrice de chacune des particules du matériau selon l'invention. La basicité de la solution est avantageusement assurée par la basicité de l'agent structurant employé ou bien par basification du mélange réactionnel par l'ajout d'un composé basique, par exemple un hydroxyde de métal alcalin, de préférence de l'hydroxyde de sodium. Le mélange réactionnel peut être mis sous conditions hydrothermales sous une pression autogène, éventuellement en ajoutant un gaz, par exemple de l'azote, à une température comprise entre la température ambiante et 200°C, de préférence entre la température ambiante et 170°C et de manière encore préférentielle à une température qui ne dépasse pas 120°C jusqu'à la formation d'une solution limpide contenant les éléments précurseurs des germes zéolithiques constituant les parois amorphes de la matrice de chacune des particules sphériques du matériau selon l'invention. Selon un mode opératoire préféré, le mélange réactionnel renfermant au moins un agent structurant, au moins un précurseur silicique et éventuellement au moins un précurseur d'au moins un élément X choisi parmi l'aluminium, le fer, le bore, l'indium et le gallium est mûri à température ambiante de façon à obtenir une solution limpide contenant les éléments précurseurs de germes de zéolithes susceptibles de générer la formation d'entités zéolithiques cristallisées.

Conformément à l'étape a) du procédé selon l'invention, les éléments précurseurs des germes zéolithiques présents dans la solution limpide sont synthétisés selon des protocoles opératoires connus de l'Homme du métier. En particulier, pour un matériau selon l'invention dont la matrice de chaque particule sphérique est constituée de germes de zéolithe bêta, une solution limpide contenant des éléments précurseurs de germes de zéolithe bêta est réalisée à partir du protocole opératoire décrit par P. Prokesova, S. Mintova, J. Cejka, T. Bein et coll., Micropor. Mesopor. Mater., 2003, 64, 165. Pour un matériau selon l'invention dont la matrice de chaque particule sphérique est constituée de germes de zéolithe Y, une solution limpide contenant des éléments précurseurs de germes de zéolithe Y est réalisée à partir du protocole opératoire décrit par Y. Liu, W. Z. Zhang, T. J. Pinnavaia et coll., J. Am. Chem. Soc., 2000, 122, 8791. Pour un matériau selon l'invention dont la matrice de chaque particule sphérique est constituée de germes de zéolithe faujasite, une solution limpide contenant des éléments précurseurs de germes de zéolithe faujasite est réalisée à partir du protocole opératoire décrit par K. R. Kloetstra, H. W. Zandbergen, J. C. Jansen, H. vanBekkum, Microporous Mater., 1996, 6, 287. Pour un matériau selon l'invention dont la matrice de chaque particule sphérique est constituée de germes de zéolithe ZSM-5, une solution limpide contenant des éléments précurseurs de germes de zéolithe ZSM-5 est réalisée à partir du protocole opératoire décrit par A. E. Persson, B. J. Schoeman, J. Sterte, J. -E. Otterstedt, Zeolites, 1995, 15, 611, le protocole opératoire exact faisant l'objet de l'exemple 1 de la présente demande. Dans le cas particulier d'un matériau purement silicique, la solution limpide contenant les éléments précurseurs de germes de zéolithe silicalite constitutives des parois du matériau selon l'invention est avantageusement réalisée à partir du protocole opératoire décrit par A. E. Persson, B. J. Schoeman, J. Sterte, J. -E. Otterstedt, Zeolites, 1994, 14, 557.

Conformément à l'étape b) du procédé de préparation du matériau selon l'invention, le tensioactif utilisé est un tensioactif ionique ou non ionique ou un mélange des deux. De préférence, le tensioactif ionique est choisi parmi des tensioactifs anioniques tels que les sulfates comme par exemple le dodécylsulfate de sodium (SDS). De préférence, le tensioactif non ionique peut être tout copolymère possédant au moins deux parties de polarités différentes leur conférant des propriétés de macromolécules amphiphiles. Ces copolymères peuvent faire partie de la liste non exhaustive des familles de copolymères suivantes : les copolymères fluorés (-[CH₂-CH₂-CH₂-CH₂-O-CO-R1- avec R1 = C₄F₉, C₈F₁₇, etc.), les copolymères biologiques comme les polyacides aminés (poly-lysine, alginates, etc.), les dendrimères, les copolymères bloc constitués de chaînes de poly(oxyde d'alkylène) et tout autre copolymère à caractère amphiphile connu de l'Homme du métier (S. Förster, M. Antionnetti, Adv.Mater, 1998, 10, 195-217, S. Fôrster, T.Plantenberg, Angew. Chem. Int. Ed, 2002, 41, 688-714, H. Cölfen, Macromol. Rapid Commun, 2001, 22, 219-252).

De manière préférée, on utilise dans le cadre de la présente invention un copolymère bloc constitué de chaînes de poly(oxyde d'alkylène). Ledit copolymère bloc est de préférence un copolymère bloc ayant deux, trois ou quatre blocs, chaque bloc étant constitué d'une chaîne de poly(oxyde d'alkylène). Pour un copolymère à deux blocs, l'un des blocs est constitué d'une chaîne de poly(oxyde d'alkylène) de nature hydrophile et l'autre bloc est constitué d'une chaîne de poly(oxyde d'alkylène) de nature hydrophobe. Pour un copolymère à trois blocs, deux des blocs sont constitués d'une chaîne de poly(oxyde d'alkylène) de nature hydrophile tandis que l'autre bloc, situé entre les deux blocs aux parties hydrophiles, est constitué d'une chaîne de poly(oxyde d'alkylène) de nature hydrophobe. De préférence, dans le cas d'un copolymère à trois blocs, les chaînes de poly(oxyde d'alkylène) de nature hydrophile sont des chaînes de poly(oxyde d'éthylène) notées (PEO)ₓ et (PEO)_{z} et les chaînes de poly(oxyde d'alkylène) de nature hydrophobe sont des chaînes de poly(oxyde de propylène) notées (PPO)y, des chaînes de poly(oxyde de butylène), ou des chaînes mixtes dont chaque chaîne est un mélange de plusieurs monomères d'oxyde d'alkylène. De manière très préférée, dans le cas d'un copolymère à trois blocs, on utilise un composé constitué de deux chaînes de poly(oxyde d'éthylène) et d'une chaîne de poly(oxyde de propylène) et plus particulièrement, il s'agit d'un composé de formule (PEO)ₓ-(PPO)_{y}-(PEO)_{z} où x est compris entre 5 et 300 et y est compris entre 33 et 300 et z est compris entre 5 et 300. De préférence, les valeurs de x et z sont identiques. On utilise très avantageusement un composé dans lequel x = 20, y = 70 et z = 20 (P123) et un composé dans lequel x = 106, y = 70 et z = 106 (F127). Les tensioactifs non-ioniques commerciaux connus sous le nom de Pluronic (BASF), Tetronic (BASF), Triton (Sigma), Tergitol (Union Carbide), Brij (Aldrich) sont utilisables en tant que tensioactifs non-ioniques dans l'étape b) du procédé de préparation de l'invention. Pour un copolymère à quatre blocs, deux des blocs sont constitués d'une chaîne de poly(oxyde d'alkylène) de nature hydrophile et les deux autres blocs sont constitués d'une chaîne de poly(oxyde d'alkylène) de nature hydrophobe.

La solution obtenue à l'issue de l'étape b) du procédé de préparation du matériau selon l'invention dans laquelle sont mélangés au moins ledit tensioactif et au moins ladite solution limpide obtenue selon l'étape a) peut être acide, neutre ou basique. De préférence, ladite solution est basique et présente de préférence un pH supérieur à 9, cette valeur du pH étant généralement imposée par le pH de la solution limpide contenant les éléments précurseurs de germes zéolithiques obtenue selon l'étape a) du procédé de préparation selon l'invention. La solution obtenue à l'issue de l'étape b) peut être aqueuse ou peut être un mélange eau-solvant organique, le solvant organique étant préférentiellement un solvant plaire, notamment un alcool, préférentiellement de l'éthanol.

La quantité en tensioactif introduit dans le mélange conformément à l'étape b) du procédé de préparation selon l'invention est définie par rapport à la quantité de matière inorganique introduite dans ledit mélange lors de l'ajout de la solution limpide contenant les éléments précurseurs de germes zéolithiques obtenue selon l'étape a) du procédé selon l'invention. La quantité de matière inorganique correspond à la quantité de matière du précurseur silicique et à celle du précurseur de l'élément X lorsqu'il est présent. Le rapport molaire n_{Inorganique} /n_{Tensioactif} est tel que le système binaire organique - inorganique formé lors de l'étape d'atomisation c) du procédé de préparation du matériau selon l'invention subisse une séparation de phase caractérisée par l'apparition d'un réseau biphasique interconnecté dont le mécanisme de formation est une décomposition spinodale. Le domaine de composition pour lequel se produit une séparation de phase par un mécanisme de décomposition spinodale est délimité par des bornes pour lesquelles l'enthalpie libre G du système binaire est minimisée (∂G/∂x = 0, x étant la fraction molaire de la phase organique, 1-x celle de la phase inorganique) et pour chaque composition appartenant à ce domaine, la dérivée seconde de l'enthalpie ∂²G/∂²x est supérieure à 0. Le principe de la séparation de phase via un mécanisme de décomposition spinodale a largement été décrit par Nakanishi pour l'obtention de gels de silice en présence de polymères (K. Nakanishi, Journal of Porous Materials, 1997, 4, 67). L'interconnexion particulière du réseau biphasique organique - inorganique résultant de ce phénomène de séparation de phase par décomposition spinodale est à l'origine de la texture mésoporeuse et macroporeuse particulière présentée par le matériau selon l'invention. Conformément à l'étape b) du procédé selon l'invention, la concentration initiale en tensioactif, introduit dans le mélange, définie par C₀ est telle que C₀ est inférieure ou égale à C_{mc}, le paramètre C_{mc} représentant la concentration micellaire critique bien connue de l'Homme du métier, c'est-à-dire la concentration limite au-delà de laquelle se produit le phénomène d'auto-arrangement des molécules du tensioactif dans la solution. Avant atomisation, la concentration en molécules de tensioactif de la solution définie par l'étape b) du procédé de préparation du matériau selon l'invention ne conduit donc pas à la formation de phases micellaires particulières. Dans une mise en oeuvre préférée du procédé selon l'invention, la concentration C₀ est inférieure à la C_{mc}, le rapport n_{Inorganique} /n_{Tensioactif} est tel que la composition du système binaire vérifie les conditions de composition pour lesquelles un mécanisme de séparation de phase se produit par décomposition spinodale et ladite solution visée à l'étape b) du procédé de préparation selon l'invention est un mélange eau basique-alcool.

L'étape d'atomisation du mélange selon l'étape c) du procédé de préparation selon l'invention produit des gouttelettes sphériques. La distribution en taille de ces gouttelettes est de type Iognormale. Le générateur d'aérosol utilisé ici est un appareil commercial de modèle 9306 A fourni par TSI ayant un atomiseur 6 jets. L'atomisation de la solution se fait dans une chambre dans laquelle est envoyé un gaz vecteur, un mélange O₂/N₂ (air sec), sous une pression P égale à 1,5 bar.

Conformément à l'étape d) du procédé de préparation selon l'invention, on procède au séchage desdites gouttelettes. Ce séchage est réalisé par le transport desdites gouttelettes via le gaz vecteur, le mélange O₂/N₂, dans des tubes en PVC, ce qui conduit à l'évaporation progressive de la solution, par exemple de la solution aquo-organique basique obtenue au cours de l'étape b) du procédé de préparation du matériau selon l'invention, et ainsi à l'obtention de particules élémentaires sphériques. Ce séchage est parfait par un passage desdites particules dans un four dont la température peut être ajustée, la gamme habituelle de température variant de 50 à 600°C et de préférence de 80 à 400°C, le temps de résidence de ces particules dans le four étant de l'ordre de la seconde. Les particules sont alors récoltées dans un filtre. Une pompe placée en fin de circuit favorise l'acheminement des espèces dans le dispositif expérimental aérosol. Le séchage des gouttelettes selon l'étape d) du procédé selon l'invention est avantageusement suivi d'un passage à l'étuve à une température comprise entre 50 et 150°C.

Dans le cas particulier où l'élément X, éventuellement utilisé pour la mise en oeuvre de l'étape a) du procédé selon l'invention, est l'élément aluminium et où l'élément sodium est présent dans la solution limpide obtenue conformément à l'étape a) du procédé selon l'invention via l'emploi de l'hydroxyde de sodium et/ou d'un agent structurant sodé assurant la basicité de ladite solution limpide, il est préféré de réaliser une étape supplémentaire d'échange ionique permettant d'échanger le cation Na⁺ par le cation NH₄⁺ entre les étapes d) et e) du procédé selon l'invention. Cet échange, qui conduit à la formation de protons H⁺ après l'étape e) du procédé selon l'invention dans le cas préféré où l'élimination de l'agent structurant et du tensioactif est réalisée par calcination sous air, est réalisé selon des protocoles opératoires bien connus de l'Homme du métier. Une des méthodes usuelles consiste à mettre en suspension les particules solides séchées issues de l'étape d) du procédé selon l'invention dans une solution aqueuse de nitrate d'ammonium. L'ensemble est ensuite porté à reflux pendant une durée de 1 à 6 heures. Les particules sont alors récupérées par filtration (centrifugation 9000 tr/mn), lavées puis séchées par passage à l'étuve à une température comprise entre 50 et 150°C. Ce cycle d'échange ionique/lavage/séchage peut être reconduit plusieurs fois et de préférence deux autres fois. Ce cycle d'échange peut être également réalisé après les étapes d) et e) du procédé selon l'invention. Dans ces conditions, l'étape e) est alors reproduite après le dernier cycle d'échange de façon à générer les protons H⁺ comme explicité ci-dessus.

Conformément à l'étape e) du procédé de préparation selon l'invention, l'élimination de l'agent structurant et du tensioactif, afin d'obtenir le matériau selon l'invention à porosité hiérarchisée, est avantageusement réalisée par des procédés d'extraction chimique ou par traitement thermique et de préférence par calcination sous air dans une gamme de température de 300 à 1000°C et plus précisément dans une gamme de 400 à 600°C pendant une durée de 1 à 24 heures et de façon préférée pendant une durée de 2 à 12 heures.

Dans le cas où la solution visée à l'étape b) du procédé de préparation selon l'invention est un mélange eau-solvant organique, de préférence basique, il est essentiel au cours de l'étape b) du procédé de préparation selon l'invention que la concentration en tensioactif soit inférieure à la concentration micellaire critique et que le rapport n_{Inorganique}/n_{Tensioactif} soit tel que la variation de l'enthalpie libre de mélange ΔGₘ et la dérivée seconde de l'enthalpie libre ∂²G/∂²x soient supérieures à 0 de sorte que l'évaporation de ladite solution aquo-organique, préférentiellement basique, au cours de l'étape c) du procédé de préparation selon l'invention par la technique d'aérosol induise un phénomène de séparation des phases organique et inorganique par décomposition spinodale conduisant à la génération des phases mésoporeuse et macroporeuse des particules sphériques du matériau selon l'invention. Ladite séparation de phase observée est consécutive à une concentration progressive, au sein de chaque gouttelette, du précurseur silicique, éventuellement du précurseur de l'élément X, de préférence du précurseur aluminique et du tensioactif résultant d'une évaporation de la solution aquo-organique, préférentiellement basique, jusqu'à une concentration en réactifs suffisante pour provoquer ledit phénomène.

Le matériau à porosité hiérarchisée de la présente invention peut être obtenu sous forme de poudre, de billes, de pastilles, de granulés, ou d'extrudés, les opérations de mises en forme étant réalisées par les techniques classiques connues de l'Homme du métier. De préférence, le matériau à porosité hiérarchisée selon l'invention est obtenu sous forme de poudre, laquelle est constituée de particules sphériques élémentaires ayant un diamètre maximal de 200 µm, ce qui facilite la diffusion éventuelle des réactifs dans le cas de l'emploi du matériau selon l'invention dans une application industrielle potentielle.

Le matériau de l'invention à porosité hiérarchisée est caractérisé par plusieurs techniques d'analyses et notamment par volumétrie à l'azote (BET), par porosimétrie au mercure, par microscopie électronique à transmission (MET), par microscopie électronique à balayage (MEB) et par fluorescence X (FX).

La volumétrie à l'azote qui correspond à l'adsorption physique de molécules d'azote dans la porosité du matériau via une augmentation progressive de la pression à température constante renseigne sur les caractéristiques texturales (diamètre de pores, type de porosité, surface spécifique) particulières du matériau selon l'invention. En particulier, elle permet d'accéder à la valeur totale du volume microporeux et mésoporeux du matériau. L'allure de l'isotherme d'adsorption d'azote et de la boucle d'hystérésis peut renseigner sur la présence de la microporosité liée aux germes zéolithiques constituant les parois amorphes de la matrice de chacune des particules sphériques du matériau selon l'invention et sur la nature de la mésoporosité. L'analyse quantitative de la microporosité du matériau selon l'invention est effectuée à partir des méthodes "t" (méthode de Lippens-De Boer, 1965) ou "αₛ" (méthode proposée par Sing) qui correspondent à des transformées de l'isotherme d'adsorption de départ comme décrit dans l'ouvrage *"*Adsorption by powders and porous solids. Principles, methodology and applications" écrit par F. Rouquerol, J. Rouquerol et K. Sing, Academic Press, 1999. Ces méthodes permettent d'accéder en particulier à la valeur du volume microporeux caractéristique de la microporosité du matériau selon l'invention ainsi qu'à la surface spécifique de l'échantillon. Le solide de référence utilisé est une silice LiChrospher Si-1000 (M. Jaroniec, M. Kruck, J. P. Olivier, Langmuir, 1999, 15, 5410). Pour exemple, l'isotherme d'adsorption d'azote d'un matériau à porosité mésoporeuse et macroporeuse, dont les parois microporeuses de la matrice de chacune des particules sphériques sont constituées de germes de zéolithe ZSM-5 (MFI), obtenu selon le procédé de préparation du matériau selon l'invention en utilisant du TEOS comme précurseur silicique, Al(O^{s}C₄H₉)₃ comme précurseur aluminique, TPAOH comme agent structurant et le copolymère à bloc particulier dénommé poly(oxyde d'éthytène)₁₀₆-poly(oxyde de propylène)₇₀-Poly(oxyde d'éthylène)₁₀₆ (PEO₁₀₆-PPO₇₀-PEO₁₀₆ ou F127) comme tensioactif présente un saut d'adsorption élevé dans le domaine des faibles valeurs de P/P0 (où P0 est la pression de vapeur saturante à la température T) suivi d'un plateau de très faible pente sur un grand domaine de pression, caractéristiques d'un matériau microporeux ainsi qu'une isotherme de type IV et une boucle d'hystérésis de type H1 dans le domaine des valeurs élevées de P/P0 représentatives de mésopores dont la taille est comprise dans une gamme de 2 à 50 nm. De même, la courbe V_{ads} (ml/g) = f(αₛ) obtenue via la méthode αₛ citée ci-dessus est caractéristique de la présence de microporosité au sein du matériau et conduit à une valeur du volume microporeux comprise dans une gamme de 0,03 à 0,4 ml/g. La détermination du volume microporeux et mésoporeux total et du volume microporeux comme décrite ci-dessus conduit à une valeur du volume mésoporeux du matériau selon l'invention dans une gamme de 0,01 à 1 ml/g.

L'analyse porosimétrie au mercure correspond à l'intrusion d'un volume de mercure caractéristique de l'existence de mésopores et de macropores dans le matériau selon l'invention selon la norme ASTM D4284-83 à une pression maximale de 4000 bars, utilisant une tension de surface de 484 dyne/cm, et un angle de contact de 140° (valeur choisie suivant les recommandations de l'ouvrage "Technique de l'ingénieur, traité analyse et caractérisation", page 1050, écrit par J. Charpin et B. Rasneur) et les pores étant supposés de forme cylindrique. Cette technique est parfaitement adaptée à l'analyse d'échantillons mésoporeux et macroporeux en complément de la technique d'analyse par volumétrie à l'azote décrite ci-dessus. En particulier, cette technique permet d'accéder à la valeur du volume mercure mésoporeux (V_{Hgméso} en ml/g) défini comme étant le volume mercure adsorbé par l'ensemble des pores ayant un diamètre compris dans la gamme des mésopores à savoir compris entre 3,6 nm et 50 nm (valeur de la borne supérieure comme définie selon la norme IUPAC). De même, le volume mercure macroporeux (V_{Hgmacro} en ml/g) est défini comme étant le volume mercure adsorbé par l'ensemble des pores ayant un diamètre supérieur ou égal à 50 nm. Pour exemple, l'analyse par porosimétrie au mercure d'un matériau à porosité mésoporeuse et macroporeuse, dont les parois microporeuses de la matrice de chacune des particules sphériques sont constituées de germes de zéolithe ZSM-5 (MFI), obtenu selon le procédé de l'invention en utilisant du TEOS comme précurseur silicique, Al(O^{s}C₄H₉)₃ comme précurseur aluminique, TPAOH comme agent structurant et le copolymère à bloc particulier dénommé poly(oxyde d'éthylène)₁₀₆-poly(oxyde de propylène)₇₀-poly(oxyde d'éthylène)₁₀₆ (PEO₁₀₆-PPO₇₀-PE0₁₀₆ ou F127) comme tensioactif conduit à un volume mercure mésoporeux compris dans une gamme de 0,01 à 1 ml/g et à un volume mercure macroporeux compris dans une gamme de 0,05 à 1 ml/g.

L'analyse par microscopie électronique par transmission (MET) est une technique également largement utilisée pour caractériser la mésoporosité et la macroporosité du matériau selon l'invention. Celle-ci permet la formation d'une image du solide étudié, les contrastes observés étant caractéristiques de l'organisation structurale, de la texture, de la morphologie ou bien de la composition chimique des particules observées, la résolution de la technique atteignant au maximum 0,2 nm. Dans l'exposé qui suit, les photos MET seront réalisées à partir de sections microtomes de l'échantillon afin de visualiser une section d'une particule sphérique élémentaire du matériau selon l'invention. Pour exemple, les images MET obtenues pour un matériau à porosité mésoporeuse et macroporeuse dont les parois microporeuses sont constituées de germes de zéolithe ZSM-5 (MFI) obtenu selon le procédé de préparation selon l'invention en utilisant du TEOS comme précurseur silicique, Al(O^{s}C₄H₉)₃ comme précurseur aluminique, TPAOH comme agent structurant et le copolymère à bloc particulier dénommé poly(oxyde d'éthylène)₁₀₆-poly(oxyde de propylène)₇₀-poly(oxyde d'éthylène)₁₀₆ (PEO₁₀₆-PPO₇₀-PEO₁₀₆ ou F127) comme tensioactif présentent au sein d'une même particule sphérique une mésoporosité et une macroporosité caractéristiques d'une séparation de phase organique - inorganique par un mécanisme de décomposition spinodale consécutive à l'étape c) d'atomisation du procédé de préparation du matériau selon l'invention dont la taille des domaines est respectivement comprise dans une gamme de 15 à 50 nm et dans une gamme de 100 à 400 nm.

La morphologie et la distribution en taille des particules élémentaires ont été établies par analyse de photos obtenues par microscopie électronique à balayage (MEB).

La présente invention concerne aussi l'utilisation du matériau à porosité hiérarchisé selon l'invention comme adsorbant pour le contrôle de la pollution ou comme tamis moléculaire pour la séparation. La présente invention a donc également pour objet un adsorbant comprenant le matériau à porosité hiérarchisé selon l'invention. Il est également avantageusement utilisé comme catalyseurs, notamment comme catalyseurs acides, de réactions par exemple celles intervenant dans les domaines du raffinage et de la pétrochimie.

Lorsque le matériau à porosité hiérarchisé selon l'invention est utilisé comme catalyseur, ce matériau peut être associé à une matrice inorganique qui peut être inerte ou catalytiquement active et à une phase métallique. La matrice inorganique peut être présente simplement comme liant pour maintenir ensemble les particules dudit matériau sous les différentes formes connues des catalyseurs (extrudés, pastilles, billes, poudres) ou bien peut être ajoutée comme diluant pour imposer le degré de conversion dans un procédé qui sinon progresserait à une allure trop rapide, conduisant à un encrassement du catalyseur en conséquence d'une formation importante de coke. Des matrices inorganiques typiques sont notamment des matières de support pour les catalyseurs comme les différentes formes de silice, d'alumine, de silice-alumine, la magnésie, la zircone, les oxydes de titane, de bore, les phosphates d'aluminium, de titane, de zirconium, les argiles telles que le kaolin, la bentonite, la montmorillonite, la sépiolite, l'attapulgite, la terre à foulon, les matières poreuses synthétiques comme SiO₂-AL₂O₃, SiO₂-ZrO₂, SiO₂-ThO₂, SiO₂-BeO, SiO₂-TiO₂ ou toute combinaison de ces composés. La matrice inorganique peut être un mélange de différents composés, en particulier d'une phase inerte et d'une phase active. Ledit matériau de la présente invention peut aussi être associé à au moins une zéolithe et jouer le rôle de phase active principale ou d'additif. La phase métallique peut être introduite intégralement sur ledit matériau de l'invention. Elle peut être également introduite intégralement sur la matrice inorganique ou encore sur l'ensemble matrice inorganique - matériau à porosité hiérarchisée par échange d'ions ou imprégnation avec des cations ou oxydes choisis parmi les éléments suivants : Cu, Ag, Ga, Mg, Ca, Sr, Zn, Cd, B, Al, Sn, Pb, V, P, Sb, Cr, Mo, W, Mn, Re, Fe, Co, Ni, Pt, Pd, Ru, Rh, Os, Ir et tout autre élément de la classification périodique des éléments.

Les compositions catalytiques comportant le matériau de la présente invention conviennent de façon générale à la mise en oeuvre des principaux procédés de transformation des hydrocarbures et des réactions de synthèse de composés organiques.

Les compositions catalytiques comportant le matériau de l'invention trouvent avantageusement leur application dans les réactions d'isomérisation, de transalkylation et de dismutation, d'alkylation et de désalkylation, d'hydratation et de déshydratation, d'oligomérisation et de polymérisation, de cyclisation, d'aromatisation, de craquage, de reformage, d'hydrogénation et de déshydrogénation, d'oxydation, d'halogénation, d'hydrocraquage, d'hydroconversion, d'hydrotraitement, d'hydrodésulfuration et d'hydrodéazotation, d'élimination catalytique des oxydes d'azote, les dites réactions impliquant des charges comprenant des hydrocarbures aliphatiques saturés et insaturés, des hydrocarbures aromatiques, des composés organiques oxygénés et des composés organiques contenant de l'azote et/ou du soufre ainsi que des composés organiques contenant d'autres groupes fonctionnels. De manière très préférée, le matériau selon l'invention est utilisé comme catalyseur dans des réactions de craquage catalytique de molécules hydrocarbonées, notamment du cumène. Ces réactions de craquage sont réalisées à une température comprise entre 150 et 450°C, à pression atmosphérique et avec un débit de gaz vecteur (par exemple N₂) compris dans une gamme de 24 à 72 l/h/g de catalyseur. Le gaz vecteur peut également être de l'hélium. De façon préférée, le flux du gaz vecteur N₂ est de 45 à 65 l/h/g de catalyseur et la pression partielle de cumène est constante et de préférence comprise entre 650 et 850 Pa. S'agissant du craquage du cumène le matériau selon l'invention réalise de façon optimale la conversion du cumène en benzène et propène.

L'invention est illustrée au moyen des exemples suivants.

### EXEMPLES

Dans les exemples qui suivent, la technique aérosol utilisée est celle décrite ci-dessus dans l'exposé de l'invention.

Pour chacun des exemples 1 à 5 ci-dessous, on calcule le rapport V_{inorganique}/V_{organique} du mélange issu de l'étape b). Ce rapport est défini comme suit : V _{inorganique}/V_{organique} = (m_{inorg} * ρ_{org}) / (m_{org} *ρ_{inorg}) avec m_{inorg} la masse finale de la fraction inorganique sous forme d'oxyde(s) condensé(s), à savoir SiO₂ et AlO₂, dans la particule élémentaire solide, m_{org} la masse totale de la faction organique non volatile se retrouvant dans la particule élémentaire solide, à savoir le tensioactif et l'agent structurant, ρ_{org} et ρ_{inorg} les densités respectivement associées aux fractions organique non volatile et inorganique. Dans les exemples qui suivent, on considère que ρ_{org} = 1 et ρ_{inorg} = 2. Aussi le rapport V_{inorganique}/V_{organique} est calculé comme étant égal à V_{inorganique}/V_{organique} = (m_{SiO2} + m_{AlO2}) / [2*(m_{agent} structurant + m_{tensioactif})]. L'éthanol, la soude, l'eau n'entrent pas en compte dans le calcul dudit rapport V_{inorganique}/V_{organique.}

Exemple 1 (invention) : Préparation d'un matériau à porosité mésoporeuse et macroporeuse dont les parois amorphes microporeuses sont constituées de germes de zéolithe ZSM-5 (MFI) tel que le rapport molaire Si/Al = 10.

10,05 g d'une solution d'hydroxyde de tétrapropylammonium (TPAOH 20% en masse dans une solution aqueuse) sont ajoutés à 4,3 g d'eau déminéralisée et 9,2 mg d'hydroxyde de sodium NaOH. Le tout est laissé sous agitation pendant 10 minutes. 0,7 g de sec-butoxyde d'aluminium (Al(O^{s}C₄H₉)₃) sont alors introduits. L'hydrolyse du précurseur aluminique est réalisée pendant 1 heure. 6 g de tétraéthylorthosilicate (TEOS) sont alors ajoutés. Le tout est maintenu sous agitation pendant 18 heures à température ambiante de manière à obtenir une solution limpide. 18 ml de cette solution sont alors ajoutés à une solution contenant 35,2 g d'éthanol, 11,3 g d'eau et 2 g de tensioactif F127 (pH du mélange = 10,5). Le rapport V_{inorganique}/V_{organique} du mélange est égal à 0,21 et est calculé comme décrit ci-dessus. Le tout est laissé sous agitation pendant 10 minutes. L'ensemble est envoyé dans la chambre d'atomisation du générateur d'aérosol tel qu'il a été décrit dans la description ci-dessus et la solution est pulvérisée sous la forme de fines gouttelettes sous l'action du gaz vecteur (air sec) introduit sous pression (P = 1,5 bar). Les gouttelettes sont séchées selon le protocole décrit dans l'exposé de l'invention ci-dessus : elles sont acheminées via un mélange O₂/N₂ dans des tubes en PVC. Elles sont ensuite introduites dans un four réglé à une température de séchage fixée à 250°C. La poudre récoltée est alors séchée 12 heures à l'étuve à 95°C. La poudre est alors calcinée sous air pendant 5 h à 550°C. La poudre ainsi obtenue est alors mise en suspension dans une solution aqueuse de NH₄NO₃ (1 mol/l) pendant 2 h 30 à température ambiante puis pendant 1 heure à 60°C sous agitation. Après filtration (centrifugation 9000 tr/min) et lavages à l'eau déminéralisée, la poudre est à nouveau séchée à l'étuve à 60°C et calcinée sous air pendant 5 h à 550°C. Le solide est caractérisé par volumétrie à l'azote, par porosimétrie au mercure, par MET, par MEB, par FX. L'analyse MET montre que les particules sphériques constitutives du matériau présentent une macroporosité de coeur caractérisée par des domaines de 300 à 500 nm de long et de 100 à 200 nm de large et une mésoporosité en périphérie des particules caractérisée par des domaines de 20 à 50 nm, l'ensemble étant caractéristique d'une séparation de phase organique - inorganique obtenue par un mécanisme de décomposition spinodale présente avant l'étape de calcination. L'analyse par volumétrie à l'azote combinée à l'analyse par la méthode αₛ conduit à une valeur du volume microporeux V_{micro} (N₂) de 0,04 ml/g, une valeur du volume mésoporeux V_{méso} (N₂) de 0,23 ml/g et une surface spécifique du matériau final de S = 178 m²/g. Le volume mercure macroporeux défini par porosimétrie au mercure est de 0,20 ml/g (la valeur du volume mercure mésoporeux également obtenue par porosimétrie au mercure est en parfait accord avec la valeur obtenue par volumétrie à l'azote). Le rapport molaire Si/Al obtenu par FX est de 10. Un cliché MEB des particules élémentaires sphériques ainsi obtenues indique que ces particules ont une taille caractérisée par un diamètre variant de 50 à 700 nm, la distribution en taille de ces particules étant centrée autour de 300 nm.

Exemple 2 (invention) : Préparation d'un matériau à porosité mésoporeuse et macroporeuse dont les parois amorphes microporeuses sont constituées de germes de zéolithe ZSM-5 (MFI) tel que le rapport molaire Si/Al = 4.

10,05 g d'une solution d'hydroxyde de tétrapropylammonium (TPAOH 20% en masse dans une solution aqueuse) sont ajoutés à 4,3 g d'eau déminéralisée et 9,2 mg d'hydroxyde de sodium NaOH. Le tout est laissé sous agitation pendant 10 minutes. 1,75 g de sec-butoxyde d'aluminium (Al(O^{s}C₄H₉)₃) sont alors introduits. L'hydrolyse du précurseur aluminique est réalisée pendant 1 heure. 6 g de tétraéthylorthosilicate (TEOS) sont alors ajoutés. Le tout est maintenu sous agitation pendant 18 heures à température ambiante de manière à obtenir une solution limpide. 18 ml de cette solution sont alors ajoutés à une solution contenant 35,2 g d'éthanol, 11,3 g d'eau et 2 g de tensioactif F127 (pH du mélange = 10,5). Le rapport V_{inorganique}/V_{organique} du mélange est égal à 0,235 et est calculé comme décrit ci-dessus. Le tout est laissé sous agitation pendant 10 minutes. L'ensemble est envoyé dans la chambre d'atomisation du générateur d'aérosol tel qu'il a été décrit dans la description ci-dessus et la solution est pulvérisée sous la forme de fines gouttelettes sous l'action du gaz vecteur (air sec) introduit sous pression (P = 1,5 bar). Les gouttelettes sont séchées selon le protocole décrit dans l'exposé de l'invention ci-dessus : elles sont acheminées via un mélange O₂/N₂ dans des tubes en PVC. Elles sont ensuite introduites dans un four réglé à une température de séchage fixée à 250°C. La poudre récoltée est alors séchée 12 heures à l'étuve à 95°C. La poudre est alors calcinée sous air pendant 5 h à 550°C. La poudre ainsi obtenue est alors mise en suspension dans une solution aqueuse de NH₄NO₃ (1 mol/l) pendant 2 h 30 à température ambiante puis pendant 1 heure à 60°C sous agitation. Après filtration (centrifugation 9000 tr/min) et lavages à l'eau déminéralisée, la poudre est à nouveau séchée à l'étuve à 60°C et calcinée sous air pendant 5 h à 550°C. Le solide est caractérisé par volumétrie à l'azote, par porosimétrie au mercure, par MET, par MEB, par FX. L'analyse MET montre que les particules sphériques constitutives du matériau présentent une macroporosité de coeur caractérisée par des domaines de 300 à 500 nm de long et de 100 à 200 nm de large et une mésoporosité en périphérie des particules caractérisée par des domaines de 20 à 50 nm, l'ensemble étant caractéristique d'une séparation de phase organique - inorganique obtenue par un mécanisme de décomposition spinodale présente avant l'étape de calcination. L'analyse par volumétrie à l'azote combinée à l'analyse par la méthode αₛ conduit à une valeur du volume microporeux V_{micro} (N₂) de 0,03 ml/g; une valeur du volume mésoporeux V_{méso} (N₂) de 0,19 ml/g et une surface spécifique du matériau final de S = 130 m²/g. Le volume mercure macroporeux défini par porosimétrie au mercure est de 0,13 ml/g (la valeur du volume mercure mésoporeux également obtenue par porosimétrie au mercure est en parfait accord avec la valeur obtenue par volumétrie à l'azote). Le rapport molaire Si/Al obtenu par FX est de 4. Un cliché MEB des particules élémentaires sphériques ainsi obtenues indique que ces particules ont une taille caractérisée par un diamètre variant de 50 à 700 nm, la distribution en taille de ces particules étant centrée autour de 300 nm.

Exemple 3 (invention) : Préparation d'un matériau à porosité mésoporeuse et macroporeuse dont les parois amorphes microporeuses sont constituées de germes de silicalite (MFI).

10,05 g d'une solution d'hydroxyde de tétrapropylammonium (TPAOH 20% en masse dans une solution aqueuse) sont ajoutés à 4,3 g d'eau déminéralisée et 9,2 mg d'hydroxyde de sodium NaOH. Le tout est laissé sous agitation pendant 10 minutes. 6 g de tétraéthylorthosilicate (TEOS) sont alors ajoutés. Le tout est maintenu sous agitation pendant 18 heures à température ambiante de manière à obtenir une solution limpide. 18 ml de cette solution sont alors ajoutés à une solution contenant 35,2 g d'éthanol, 11,3 g d'eau et 2 g de tensioactif F127 (pH du mélange = 10,5). Le rapport V_{inorganique}/V_{organique} du mélange est égal à 0,20 et est calculé comme décrit ci-dessus. Le tout est laissé sous agitation pendant 10 minutes. L'ensemble est envoyé dans la chambre d'atomisation du générateur d'aérosol tel qu'il a été décrit dans la description ci-dessus et la solution est pulvérisée sous la forme de fines gouttelettes sous l'action du gaz vecteur (air sec) introduit sous pression (P = 1,5 bar). Les gouttelettes sont séchées selon le protocole décrit dans l'exposé de l'invention ci-dessus : elles sont acheminées via un mélange O₂/N₂ dans des tubes en PVC. Elles sont ensuite introduites dans un four réglé à une température de séchage fixée à 250°C. La poudre récoltée est alors séchée 12 heures à l'étuve à 95°C. La poudre est alors calcinée sous air pendant 5 h à 550°C. Le solide est caractérisé par volumétrie à l'azote, par porosimétrie au mercure, par MET, par MEB. L'analyse MET montre que les particules sphériques constitutives du matériau présentent une macroporosité de coeur caractérisée par des domaines de 300 à 500 nm de long et de 100 à 200 nm de large et une mésoporosité en périphérie des particules caractérisée par des domaines de 20 à 50 nm, l'ensemble étant caractéristique d'une séparation de phase organique - inorganique obtenue par un mécanisme de décomposition spinodale présente avant l'étape de calcination. L'analyse par volumétrie à l'azote combinée à l'analyse par la méthode αₛ conduit à une valeur du volume microporeux V_{micro} de 0,3 ml/g (N₂), une valeur du volume mésoporeux V_{méso} de 0,5 ml/g (N₂) et une surface spécifique du matériau final de S = 620 m²/g. Le volume mercure macroporeux défini par porosimétrie au mercure est de 0,4 ml/g (la valeur du volume mercure mésoporeux également obtenue par porosimétrie au mercure est en parfait accord avec la valeur obtenue par volumétrie à l'azote). Un cliché MEB des particules élémentaires sphériques ainsi obtenues indique que ces particules ont une taille caractérisée par un diamètre variant de 50 à 700 nm, la distribution en taille de ces particules étant centrée autour de 300 nm.

Exemple 4 (invention) : Préparation d'un matériau à porosité mésoporeuse et macroporeuse dont les parois amorphes microporeuses sont constituées de germes de zéolithe bêta (BEA) tel que le rapport molaire Si/Al = 50.

11,70 g d'une solution d'hydroxyde de tétraéthylammonium (TEAOH 20% en masse dans une solution aqueuse) sont ajoutés à 7,8 g d'eau déminéralisée et 0,03 g d'hydroxyde de sodium NaOH. Le tout est laissé sous agitation pendant 10 minutes. 0,14 g de sec-butoxyde d'aluminium (Al(O^{S}C₄H₉)₃) sont alors introduits. Le tout est laissé sous agitation pendant 10 minutes. L'hydrolyse du précurseur aluminique est réalisée pendant 1 heure. 6 g de tétraéthylorthosilicate (TEOS) sont alors ajoutés. Le tout est maintenu sous agitation pendant 18 heures à température ambiante de manière à obtenir une solution limpide. 18 ml de cette solution sont alors ajoutés à une solution contenant 35,2 g d'éthanol, 11,3 g d'eau et 2 g de tensioactif F127 (pH du mélange = 10). Le rapport V_{inorganique}/V_{organique} du mélange est égal à 0,17 et est calculé comme décrit ci-dessus. Le tout est laissé sous agitation pendant 10 minutes. L'ensemble est envoyé dans la chambre d'atomisation du générateur d'aérosol tel qu'il a été décrit dans la description ci-dessus et la solution est pulvérisée sous la forme de fines gouttelettes sous l'action du gaz vecteur (air sec) introduit sous pression (P = 1,5 bar). Les gouttelettes sont séchées selon le protocole décrit dans l'exposé de l'invention ci-dessus : elles sont acheminées via un mélange O₂/N₂ dans des tubes en PVC. Elles sont ensuite introduites dans un four réglé à une température de séchage fixée à 250°C. La poudre récoltée est alors séchée 12 heures à l'étuve à 95°C. La poudre est alors calcinée sous air pendant 5 h à 550°C. La poudre ainsi obtenue est alors mise en suspension dans une solution aqueuse de NH₄NO₃ (1 mol/l) pendant 2 h 30 à température ambiante puis pendant 1 heure à 60°C sous agitation. Après filtration (centrifugation 9000 tr/min) et lavages à l'eau déminéralisée, la poudre est à nouveau séchée à l'étuve à 60°C et calcinée sous air pendant 5 h à 550°C. Le solide est caractérisé par volumétrie à l'azote, par porosimétrie au mercure, par MET, par MEB, par FX. L'analyse MET montre que les particules sphériques constitutives du matériau présentent une macroporosité de coeur caractérisée par des domaines de 200 à 500 nm de long et de 100 à 200 nm de large et une mésoporosité en périphérie des particules caractérisée par des domaines de 20 à 50 nm, l'ensemble étant caractéristique d'une séparation de phase organique - inorganique obtenue par un mécanisme de décomposition spinodale présente avant l'étape de calcination. L'analyse par volumétrie à l'azote combinée à l'analyse par la méthode αₛ conduit à une valeur du volume microporeux V_{micro} de 0,1 ml/g (N₂), une valeur du volume mésoporeux V_{méso} de 0,5 ml/g (N₂) et une surface spécifique du matériau final de S = 200 m²/g. Le volume mercure macroporeux défini par porosimétrie au mercure est de 0,2 ml/g (la valeur du volume mercure mésoporeux également obtenue est en parfait accord avec la valeur obtenue par volumétrie à l'azote). Le rapport molaire Si/Al obtenu par FX est de 50. Un cliché MEB des particules élémentaires sphériques ainsi obtenues indique que ces particules ont une taille caractérisée par un diamètre variant de 50 à 700 nm, la distribution en taille de ces particules étant centrée autour de 300 nm.

### Exemple 5 : Préparation d'un matériau à porosité mésoporeuse et macroporeuse dont les parois amorphes microporeuses sont constituées de germes de zéolithe Y (FAU) tel que le rapport molaire Si/Al = 8.

0,41 g d'aluminate de sodium (NaAlO₂) sont ajoutés à une solution contenant 0,17 g d'hydroxyde de sodium et 7,6 g d'eau déminéralisée. La solution est laissée sous agitation jusqu'à la dissolution du précurseur d'aluminium. 10 g de silicate de sodium (27% en poids en SiO₂ et 14 % en NaOH) sont alors ajoutés sous vigoureuse agitation. Le tout est maintenu sous agitation pendant 18 heures à température ambiante de manière à obtenir une solution limpide. 15 ml de cette solution sont alors ajoutés à une solution contenant 35,2 g d'éthanol, 11,6 g d'eau et 6,3 g de tensioactif F127 (pH du mélange = 9,8). Le rapport \/_{inorganique}/V_{organique} du mélange est égal à 0,24 et est calculé comme décrit ci-dessus. Le tout est laissé sous agitation pendant 10 minutes. L'ensemble est envoyé dans la chambre d'atomisation du générateur d'aérosol tel qu'il a été décrit dans la description ci-dessus et la solution est pulvérisée sous la forme de fines gouttelettes sous l'action du gaz vecteur (air sec) introduit sous pression (P = 1,5 bar). Les gouttelettes sont séchées selon le protocole décrit dans l'exposé de l'invention ci-dessus : elles sont acheminées via un mélange O₂/N₂ dans des tubes en PVC. Elles sont ensuite introduites dans un four réglé à une température de séchage fixée à 250°C. La poudre récoltée est alors séchée 12 heures à l'étuve à 95°C. La poudre est alors calcinée sous air pendant 5 h à 550°C. La poudre ainsi obtenue est alors mise en suspension dans une solution aqueuse de NH₄NO₃ (1 mol/l) pendant 2 h 30 à température ambiante puis pendant 1 heure à 60°C sous agitation. Après filtration (centrifugation 9000 tr/min) et lavages à l'eau déminéralisée, la poudre est à nouveau séchée à l'étuve à 60°C et calcinée sous air pendant 5 h à 550°C. Le solide est caractérisé par volumétrie à l'azote, par porosimétrie au mercure, par MET, par MEB, par FX. L'analyse MET montre que les particules sphériques constitutives du matériau présentent une macroporosité de coeur caractérisée par des domaines de 200 à 500 nm de long et de 100 à 200 nm de large et une mésoporosité en périphérie des particules caractérisée par des domaines de 20 à 50 nm, l'ensemble étant caractéristique d'une séparation de phase organique - inorganique obtenue par un mécanisme de décomposition spinodale présente avant l'étape de calcination. L'analyse par volumétrie à l'azote combinée à l'analyse par la méthode αₛ conduit à une valeur du volume microporeux V_{micro} de 0,25 ml/g (N₂), une valeur du volume mésoporeux V_{méso} de 0,7 ml/g (N₂) et une surface spécifique du matériau final de S = 380 m²/g. Le volume mercure macroporeux défini par porosimétrie au mercure est de 0,3 ml/g (la valeur du volume mercure mésoporeux également obtenue par porosimétrie au mercure est en parfait accord avec la valeur obtenue par volumétrie à l'azote). Le rapport molaire Si/Al obtenu par FX est de 8. Un cliché MEB des particules élémentaires sphériques ainsi obtenues indique que ces particules ont une taille caractérisée par un diamètre variant de 50 à 700 nm, la distribution en taille de ces particules étant centrée autour de 300 nm.

### Exemple 6 (invention) : Craquage du cumène catalysé par le matériau de l'exemple 1 selon l'invention.

50 mg de poudre du matériau de l'exemple 1 sont placés dans un réacteur. La poudre est alors prétraitée à 500°C pendant 2 h puis à 300°C pendant 11 h sous azote. Le cumène, maintenu à 14°C, est acheminé à travers la poudre par un flux d'azote avec un débit de 60 l/h/g de solide testé. La température de la réaction est fixée à 300°C. La réaction est effectuée sous pression atmosphérique. Les produits de la réaction sont analysés à l'aide d'un chromatographe Perichrom 2100. Le taux de conversion de la réaction de craquage du cumène en propène et benzène est fonction des propriétés d'acidité de Brönsted du solide testé. Le taux de conversion par unité de masse du matériau de l'exemple 1 est de 32% (+/-5%). Le taux de conversion par unité de surface spécifique (100 m²/g) du matériau de l'exemple 1 selon l'invention est de 18%.

### Exemple 7 (invention) : Craquage du cumène catalysé par le matériau de l'exemple 2 selon l'invention.

50 mg de poudre du matériau de l'exemple 2 sont placés dans un réacteur. La poudre est alors prétraitée à 500°C pendant 2 h puis à 300°C pendant 11 h sous azote. Le cumène, maintenu à 14°C, est acheminé à travers la poudre par un flux d'azote avec un débit de 60 l/h/g de solide testé. La température de la réaction est fixée à 300°C. La réaction est effectuée sous pression atmosphérique. Les produits de la réaction sont analysés à l'aide d'un chromatographe Perichrom 2100. Le taux de conversion de la réaction de craquage du cumène en propène et benzène est fonction des propriétés d'acidité de Brônsted du solide testé. Le taux de conversion par unité de masse du matériau de l'exemple 2 est de 37 %. Le taux de conversion par unité de surface spécifique (100 m²/g) du matériau de l'exemple 2 selon l'invention est de 30%.

### Exemple 8 (exemple comparatif) : Craquage du cumène catalysé par un aluminosilicate amorphe commercial Grace Davison, Si/Al = 4, S_{BET} = 365 m²/g.

50 mg de poudre de l'aluminosilicate commercial sont placés dans un réacteur. La poudre est alors prétraitée à 500°C pendant 2 h puis à 300°C pendant 11 h sous azote. Le cumène, maintenu à 14°C, est acheminé à travers la poudre par un flux d'azote avec un débit de 60l/h/g de solide testé. La température de la réaction est fixée à 300°C. La réaction est effectuée sous pression atmosphérique. Les produits de la réaction sont analysés à l'aide d'un chromatographe Perichrom 2100. Le taux de conversion de la réaction de craquage du cumène en propène et benzène est fonction des propriétés d'acidité de Brönsted du solide testé. Le taux de conversion par unité de masse de l'aluminosilicate commercial est de 12% (+/-5%). Le taux de conversion par unité de surface spécifique (100 m²/g) de l'aluminosilicate commercial est de 3%.

## Revendications

1. Matériau à porosité hiérarchisée constitué d'au moins deux particules sphériques élémentaires ayant un diamètre maximal de 200 microns, l'une au moins desdites particules sphériques comprend au moins une matrice à base d'oxyde de silicium et ayant des parois amorphes, ledit matériau présentant un volume macroporeux mesuré par porosimétrie au mercure compris entre 0,05 et 1 ml/g, un volume mésoporeux mesuré par volumétrie à l'azote compris entre 0,01 et 1 ml/g et un volume microporeux mesuré par volumétrie à l'azote compris entre 0,03 et 0,4 ml/g.

2. Matériau selon la revendication 1 tel que le volume macroporeux mesuré par porosimétrie au mercure est compris entre 0,1 et 0,3 ml/g.

3. Matériau selon la revendication 1 ou la revendication 2 tel que le volume mésoporeux mesuré par volumétrie à l'azote est compris entre 0,1 et 0,6 ml/g.

4. Matériau selon l'une des revendications 1 à 3 tel que la macroporosité est présente dans des domaines compris dans une gamme variant de 50 à 1000 nm.

5. Matériau selon l'une des revendications 1 à 4 tel que la mésoporosité est présente dans des domaines compris dans une gamme variant de 2 à 50 nm.

6. Matériau selon l'une des revendications 1 à 5 tel qu'il présente des particules sphériques élémentaires dépourvues de mésoporosité.

7. Matériau selon l'une des revendications 1 à 6 tel que ladite matrice présente des parois amorphes constituées de germes zéolithiques.

8. Matériau selon la revendication 7 tel que lesdits germes zéolithiques sont des espèces pour l'amorce d'au moins une zéolithe choisie parmi les zéolithes de type structural MFI, BEA, FAU et LTA.

9. Matériau selon l'une des revendications 1 à 8 tel que ladite matrice à base d'oxyde de silicium est entièrement silicique.

10. Matériau selon l'une des revendications 1 à 8 tel que ladite matrice à base d'oxyde de silicium comprend au moins un élément X choisi parmi l'aluminium, le fer, le bore, l'indium et le gallium.

11. Matériau selon la revendication 10 tel que élément X est l'aluminium.

12. Matériau selon l'une des revendications 1 à 11 tel que lesdites particules sphériques élémentaires ont un diamètre compris entre 50 nm et 10 microns.

13. Matériau selon l'une des revendications 1 à 12 tel qu'il présente une surface spécifique comprise entre 100 et 1100 m²/g.

14. Procédé de préparation d'un matériau selon l'une des revendications 1 à 13 comprenant **: a)** la préparation d'une solution limpide contenant les éléments précurseurs de germes zéolithiques, à savoir au moins un agent structurant, au moins un précurseur silicique et éventuellement au moins un précurseur d'au moins un élément X choisi parmi l'aluminium, le fer, le bore, l'indium et le gallium ; b) le mélange en solution aquo-organique d'au moins un tensioactif et d'au moins ladite solution limpide obtenue selon a), la concentration initiale en tensioactif étant inférieure à la concentration micellaire critique et la variation de l'enthalpie libre de mélange ΔGₘ ainsi que la dérivée seconde de l'enthalpie libre ∂²G/∂²x étant supérieures à 0, c) l'atomisation par aérosol de ladite solution obtenue à l'étape b) pour conduire à la formation de gouttelettes sphériques ; d) le séchage desdites gouttelettes et e) l'élimination dudit agent structurant et dudit tensioactif pour l'obtention d'un matériau amorphe à porosité hiérarchisée dans la gamme de la microporosité, de la mésoporosité et de la macroporosité.

15. Procédé selon la revendication 14 tel que élément X est l'aluminium.

16. Procédé selon la revendication 14 ou la revendication 15 tel que ledit tensioactif est un copolymère à trois blocs, chaque bloc étant constitué d'une chaîne de poly(oxyde d'alkylène).

17. Procédé selon la revendication 16 tel que ledit copolymère à trois blocs est constitué de deux chaînes de poly(oxyde d'éthylène) et d'une chaîne de poly(oxyde de propylène).

18. Adsorbant comprenant le matériau à porosité hiérarchisé selon l'une des revendications 1 à 13 ou préparé selon le procédé selon l'une des revendications 14 à 17.

19. Catalyseur comprenant le matériau à porosité hiérarchisé selon l'une des revendications 1 à 13 ou préparé selon le procédé selon l'une des revendications 14 à 17.

## Claims

1. A material with hierarchical porosity consisting of at least two elementary spherical particles having a maximum diameter of 200 microns, at least one of said spherical particles comprising at least one matrix based on silicon oxide and having amorphous walls, said material having a macropore volume measured by mercury porosimetry ranging between 0.05 and 1 ml/g, a mesopore volume measured by nitrogen volumetric analysis ranging between 0.01 and 1 ml/g and a micropore volume measured by nitrogen volumetric analysis ranging between 0.03 and 0.4 ml/g.

2. A material as claimed in claim 1, such that the macropore volume measured by mercury porosity ranges between 0.1 and 0.3 ml/g.

3. A material as claimed in claim 1 or in claim 2, such that the mesopore volume measured by nitrogen volumetric analysis ranges between 0.1 and 0.6 ml/g.

4. A material as claimed in any one of claims 1 to 3, such that the macroporosity is present in domains ranging between 50 and 1000 nm.

5. A material as claimed in any one of claims 1 to 4, such that the mesoporosity is present in domains ranging between 2 and 50 nm.

6. A material as claimed in any one of claims 1 to 5, such that it has elementary spherical particles devoid of mesoporosity.

7. A material as claimed in any one of claims 1 to 6, such that said matrix has amorphous walls consisting of zeolite seeds.

8. A material as claimed in claim 7, such that said zeolite seeds are species for priming at least one zeolite selected from among the zeolites of MFI, BEA, FAU and LTA structural type.

9. A material as claimed in any one of claims 1 to 8, such that said matrix based on silicon oxide is entirely silicic.

10. A material as claimed in any one of claims 1 to 8, such that said matrix based on silicon oxide comprises at least one element X selected from among aluminium, iron, boron, indium and gallium.

11. A material as claimed in claim 10, such that element X is aluminium.

12. A material as claimed in any one of claims 1 to 11, such that said elementary spherical particles have a diameter ranging between 50 nm and 10 microns.

13. A material as claimed in any one of claims 1 to 12, such that it has a specific surface area ranging between 100 and 1100 m²/g.

14. A method of preparing a material as claimed in any one of claims 1 to 13, comprising: a) preparing a clear solution containing the precursor elements of zeolite seeds, i.e. at least one structuring agent, at least one silicic precursor and optionally at least one precursor of at least one element X selected from among aluminium, iron, boron, indium and gallium; b) mixing into a solution at least one surfactant and at least said clear solution obtained in stage a), the surfactant initial concentration being lower than the critical micellar concentration and the variation of the free enthalpy of mixing ΔGₘ as well as the second derivative of the free enthalpy ∂²G/∂²x being greater than 0; c) aerosol atomizing said solution obtained in stage b) so as to lead to the formation of spherical droplets; d) drying said droplets; and e) eliminating said structuring agent and said surfactant so as to obtain an amorphous material with hierarchical porosity in the microporosity, mesoporosity and macroporosity domains.

15. A method as claimed in claim 14, such that element X is aluminium.

16. A method as claimed in claim 14 or in claim 15, such that said surfactant is a three-block copolymer, each block consisting of a poly(alkylene oxide) chain.

17. A method as claimed in claim 16, such that said three-block copolymer consists of two poly(ethylene oxide) chains and of one poly(propylene oxide) chain.

18. An adsorbent comprising the material with hierarchical porosity as claimed in any one of claims 1 to 13 or prepared according to the method as claimed in any one of claims 14 to 17.

19. A catalyst comprising the material with hierarchical porosity as claimed in any one of claims 1 to 13 or prepared according to the method as claimed in any one of claims 14 to 17.

## Patentansprüche

1. Werkstoff mit abgestufter Porenbeschaffenheit, der aus mindestens zwei kugelförmigen Elementarpartikeln mit einem Durchmesser von höchstens 200 Mikrometern besteht, wobei mindestens einer dieser kugelförmigen Partikel mindestens eine Matrix umfasst, die auf Siliciumoxid basiert und amorphe Wände hat, wobei der Werkstoff ein Makroporenvolumen, gemessen mittels Quecksilberporosimetrie, im Bereich von 0,05 bis 1 ml/g und ein Mesoporenvolumen, gemessen mittels Stickstoffporosimetrie, im Bereich von 0,01 bis 1 ml/g und ein Mikroporenvolumen, gemessen mittels Stickstoffporosimetrie, im Bereich von 0,03 bis 0,4 ml/g aufweist.

2. Werkstoff nach Anspruch 1, derart, dass das Makroporenvolumen, gemessen mittels Quecksilberporosimetrie, im Bereich von 0,1 bis 0,3 ml/g liegt.

3. Werkstoff nach Anspruch 1 oder Anspruch 2, derart, dass das Mesoporenvolumen, gemessen mittels Stickstoffporosimetrie, im Bereich von 0,1 bis 0,6 ml/g liegt.

4. Werkstoff nach einem der Ansprüche 1 bis 3, derart, dass die Gesamtheit der Makroporen Größenbereiche abdeckt, die zwischen 50 und 1000 nm einschließlich liegen.

5. Werkstoff nach einem der Ansprüche 1 bis 4, derart, dass die Gesamtheit der Mesoporen Größenbereiche abdeckt, die zwischen 2 und 50 nm einschließlich liegen.

6. Werkstoff nach einem der Ansprüche 1 bis 5, derart, dass die kugelförmigen Elementarpartikel keinerlei Mesoporen aufweisen.

7. Werkstoff nach einem der Ansprüche 1 bis 6, derart, dass die Matrix amorphe Wände aufweist, die aus Zeolithkeimen bestehen.

8. Werkstoff nach Anspruch 7, derart, dass es sich bei den Zeolithkeimen um Spezies handelt, die Ausgangspunkte für die Bildung mindestens eines Zeoliths darstellen, welcher aus den Zeolithen der Strukturtypen MFI, BEA, FAU und LTA ausgewählt ist.

9. Werkstoff nach einem der Ansprüche 1 bis 8, derart, dass die Matrix, die auf Siliciumoxid basiert, vollständig aus Siliciumdioxid besteht.

10. Werkstoff nach einem der Ansprüche 1 bis 8, derart, dass die Matrix, die auf Siliciumoxid basiert, mindestens ein Element X umfasst, das aus Aluminium, Eisen, Bor, Indium und Gallium ausgewählt ist.

11. Werkstoff nach Anspruch 10, derart, dass es sich bei dem Element X um Aluminium handelt.

12. Werkstoff nach einem der Ansprüche 1 bis 11, derart, dass die kugelförmigen Elementarpartikel einen Durchmesser im Bereich von 50 nm bis 10 Mikrometer haben.

13. Werkstoff nach einem der Ansprüche 1 bis 12, derart, dass er eine spezifische Oberfläche im Bereich von 100 bis 1100 m²/g aufweist.

14. Verfahren zur Herstellung eines Werkstoffs nach einem der Ansprüche 1 bis 13, umfassend: a) Herstellen einer klaren Lösung, welche die Vorläuferelemente von Zeolithkeimen enthält, nämlich mindestens ein strukturgebendes Mittel, mindestens einen Siliciumdioxidvorläufer und möglicherweise mindestens eine Vorläufersubstanz mindestens eines Elements X, das aus Aluminium, Eisen, Bor, Indium und Gallium ausgewählt ist; b) Vermischen mindestens eines Tensids und mindestens der klaren Lösung, die gemäß a) erhalten wurde, in einer wässrig-organischen Lösung, wobei die Anfangskonzentration an Tensid geringer als die kritische Micellenkonzentration ist und die Veränderung der freien Enthalpie der Mischung ΔGₘ sowie die zweite Ableitung der freien Enthalpie δ²G/δ²x größer als 0 sind, c) Zerstäuben der Lösung, die in Schritt b) erhalten wurde, als Aerosol, sodass sich kugelförmige Tröpfchen bilden; d) Trocknen der Tröpfchen und e) Entfernen des strukturgebenden Mittels und des Tensids, um einen amorphen Werkstoff zu erhalten, dessen Poren derart beschaffen sind, dass sie abgestuft in die Bereiche der Mikroporen, der Mesoporen und der Makroporen vorliegen.

15. Verfahren nach Anspruch 14, derart, dass es sich bei dem Element X um Aluminium handelt.

16. Verfahren nach Anspruch 14 oder Anspruch 15, derart, dass das Tensid ein Copolymer mit drei Blöcken ist, wobei jeder der Blöcke aus einer Poly(alkylenoxid)-Kette besteht.

17. Verfahren nach Anspruch 16, derart, dass das Copolymer mit drei Blöcken aus zwei Poly(ethylenoxid)-Ketten und einer Poly(propylenoxid)-Kette besteht.

18. Adsorptionsmittel, welches den Werkstoff mit abgestufter Porenbeschaffenheit nach einem der Ansprüche 1 bis 13, oder der nach einem der Ansprüche 14 bis 17 hergestellt ist, umfasst.

19. Katalysator, welcher den Werkstoff mit abgestufter Porenbeschaffenheit nach einem der Ansprüche 1 bis 13, oder der nach einem der Ansprüche 14 bis 17 hergestellt ist, umfasst.
